# EUROPEAN PATENT APPLICATION

(11) **EP 3 913 371 A1**
(43) Date of publication of application: **24.11.2021**
(21) Application number: 21169908.7
(22) Date of filing: 22.04.2021
(51) Int. Cl.: G01N 33/68, G01N 33/94

(54) **METHOD AND KIT FOR DIAGNOSTING IRRITABLE BOWEL SYNDROME AND FOR ITS RELIEF THROUGH DIETARY INTERVENTIONS**

(30) Priority: 18.05.2020 DE 102020113384; 02.06.2020 DE 102020114649
(71) Applicant: Neuroimmun GmbH, 76135 Karlsruhe (DE)
(72) Inventor: KELLNER, Karl-Heinz, 76135 Karlsruhe (DE)
(74) Representative: Benedum, Ulrich Max

(57) **Abstract**

Method and kit for diagnosis of irritable bowel syndrome and irritable colon, comprising: a stool extraction system for preparation of a stool extract of substances of the gut-brain-axis, a derivatizing reagent for small biogenic amines; antibodies specific for derivatized tryptophan, serotonin, and GABA; and immunoassay reagents and buffers for combined determination of tryptophan, serotonin, and GABA. The method of *in vitro* diagnosis of irritable bowel syndrome (IBS) or irritable colon comprises a collecting a specimen of fresh stool; a transfer of the specimen into a stabilizing buffer; an extraction of the biogenic amines (neurotransmitters, neuroactive substances and tissue-active substances) and substances of the gut-brain axis; a combined quantitative analysis for tryptophan, serotonin and GABA, and optionally of histamine and kynurenic acid, to determine any imbalance of substances of the gut-brain-axis and for *in vitro* diagnosis of irritable bowel syndrome and/or irritable colon based on a comparison respective values and ranges in stools of healthy subjects.

## Description

### TECHNICAL FIELD

The invention relates to the field of in vitro diagnosis of gastrointestinal disorders, and more particularly, to the use of a combination of immunological assays with stool samples as probe for the relief of undesirable gastrointestinal health problems such as irritable bowel syndrome (IBS), abdominal pain, abdominal discomfort, bloating, liquid stools, and constipation [G01N 2800/06].

### BACKGROUND

Irritable bowel syndrome (IBS) is a common disease of the gastrointestinal tract. The main symptoms are chronic abdominal pain associated with intestinal dysfunction, irregularities in defecation, i.e., diarrhea or constipation, and often stools containing mucus. Most patients also suffer from an increased gas content in the intestine, an enlarged stomach and flatulence. The most complained gastrointestinal symptoms are chronic diarrhea, bloating and abdominal pain.

Once malignancies and inflammatory bowel diseases (IBD) are excluded, irritable bowel syndrome (IBS) or IBS-like disorders, respectively, are diagnosed. Subsequent clinical diagnostics and therapies are then focused on known potential causes for IBS-like disorders and diagnosis of one or more of celiac disease, non-celiac gluten sensitivity, non-digested oligosaccharides, disaccharides, monosaccharides and polyols, lactose intolerance, small intestinal bacterial overgrowth (SIBO), α-amylase/trypsin inhibitor (ATIs) or nickel allergic contact mucositis. Pathologic conditions such as food allergies and adverse reactions to foods also receive much diagnostic attention.

The relationship between IBS as defined by Rome IV criteria (Drossman DA Functional gastrointestinal disorders: history, pathophysiology, clinical features, and Rome IV Gastroenterology 2016, 150 1262-79e) and food-related clinical conditions is obscure and subject to controversies. A pure diagnosis of IBS is not possible because of food-dependent symptoms: up to 80% of IBS patients identify food as the likely trigger for their symptoms and they are therefore asking for dietary and behavioral advice.

The prevalence of IBS or IBS-like disorders is about 10% to 20% and markedly geographically dependent: its prevalence is e.g. 21% in South America versus 7% in Southeast Asia. IBS is further twice more common in women than men. In the United States, patients are equally distributed among IBS with diarrhea (IBS-D), IBS with constipation (IBS-C), and IBS with a mixed bowel pattern (IBS-M) whereas in Europe, IBS-C (45.9%) or IBS-D (50%) are the main groups. Furthermore, the pathogenesis of IBS is heterogeneous and comprises alterations in motility, visceral sensation, brain-gut interactions, microbiome, bile acid metabolism, and intestinal permeability. An adverse immune activation is observed in case of low-grade inflammations. Colonic mucosal biopsies of patients with IBS often show dense mast cell infiltrates which are likely responsible for neuronal hyperexcitability and many IBS symptoms. Another diagnostic criterium is a leaky epithelial barrier which may lead to mast cell infiltration and activation causing IBS symptoms. On the other hand, there are no founded diagnostic tools with respect to brain-gut interactions and microbiome while 80% of the IBS patients are asking for dietary and behavioral advice.

EP3526340 A1 (Genetic Analysis AS) provides a diagnostic method for determining the likelihood that a subject with irritable bowel syndrome (IBS) will respond to treatment with an IBS intervention diet or fecal microbiota transplant (FMT). Said diagnostic method is based on analyzing the abundance of certain bacteria in gastrointestinal tract samples by nucleic acid analysis. EP3349004 A1 (Cedars-Sinai Medical Center) describes methods of diagnosing IBS by detecting the presence or absence of CDT (Cytolethal Distending Toxin of Campylobacter jejuni) or a marker similar thereto in a subject. EP3204771 A1 (Cedars-Sinai Medical Center) describes methods and systems for distinguishing IBS from IBD and coeliac disease comprising the detection of anti-vinculin antibodies and anti-CDT antibodies. WO2019/234246 A1 (4D Pharma PLC) discloses a method for stratification of patients with IBS comprising a microbiome data analysis of the "average microbiome" as well as aberrations thereof to generate a patient specific microbiome profile and for differential diagnosis of IBS. EP2780023 B1 (Multigerm UK Enterprises Ltd.) describe a probiotic operation comprising a mixture of lactobacillus and and/or enterococcus bacteria as well as a mixture of complex carbohydrates and sugars for treating IBS based on such microbiome data analyses. Casen C et al describes a diagnostic test of IBS in Deviations in human gut microbiota: a novel diagnostic test for determining dysbiosis in patients with IBS or IBD, Aliment. Pharmacol. & Therapeutics, 2015, 42(1):71-83. EP3218716 A1 (Biomerica Inc.) discloses test kits and methods comprising a selection of food preparations with established discriminatory values. EP2223121 B1 (Index Diagnostics AB) claims an *in vitro* method and kit for determining a plethora of gene expression profiles in a sample taken from a human body in a method for differentiation between inflammatory bowel disease (IBD) and irritable bowel syndrome (IBS) in a patient. EP3004892 B1 (Nestec SA) claims a method for diagnostic prediction of IBS based on a matrix of scores comprising at least one bacterial antigen marker to obtain a microbiome score, at least one mast cell marker to obtain a mast cell score, at least one inflammatory marker to obtain an inflammatory score, at least one bile acid malabsorption marker to obtain a BAM score, at least one kynurenine marker to obtain an oxidative stress score, a quantitative determination of serotonin to obtain a serotonin score; and a statistical algorithm to calculate a probability of IBS. The marker for assessing the oxidative stress score is selected from the group consisting of kynurenine, kynurenic acid, anthranilic acid, 3-hydroxy kynurenine, 3-hydroxy anthranilic acid, xanthurenic acid, quinolinic acid, tryptophan, 5-hydroxytryptophan, and combinations thereof. The genetic analyses and diagnostic scores of the prior art still carry on with an exclusion diagnostic of IBD and other gastrointestinal diseases. The result is no positive diagnosis of IBS and there is no guidance or help for the general practitioner when the patient is asking for general dietary advice and/or for dietetic intervention. The prior art represents a diagnostic problem.

### BRIEF DESCRIPTION OF THE INVENTION

The problem is ameliorated by a kit for stool specimen collection and extraction of neurotransmitters, neuroactive substances or tissue-active substances from a defined stool specimen in a buffer to obtain a defined stool extract, further comprising immunological assay for quantitative determination in said stool extract of at least three neurotransmitters, neuroactive substances or tissue-active substances of the gut-brain-axis which are either present in food or synthesized by the microbiome in gut and colon or both.

In some embodiments, the substances of the gut-brain-axis are selected from a group comprising gamma-aminobutyric acid, serotonin, and tryptophan.

In some preferred embodiments, the stool extract is further analyzed for the concentration of histamine which is a tissue-active substance present in the food as well as produced by some gastrointestinal bacteria.

In some preferred embodiments, the stool extract is further analyzed for the concentration of kynurenic acid which also possesses neuroactive activity.

Another aspect of the disclosure relates to a method of *in-vitro* diagnosis of irritable bowel syndrome (IBS) or irritable colon, comprising the steps of:-
collecting a quantitatively defined specimen of fresh stool;
transferring the stool specimen into a stool stabilizing buffer;
extracting the stool for neurotransmitters, neuroactive substances and tissue-active substances to produce a solution of neuroactive substances of the gut-brain axis;
analyzing the solution of neuroactive substance of the gut-brain axis in combination for the amounts of tryptophan, serotonin and gamma-amino butyric acid;
to determine any imbalance of neuroactive substances in stool for diagnosis of irritable bowel syndrome and/or irritable colon on the basis of cut-off-values or ranges for three or more neuroactive substances.

In some embodiments, the combined determination of tryptophan, serotonin and gamma-amino butyric acid in stool extract is by immunoassays.

In some embodiments, the combined determination of tryptophan, serotonin and gamma-amino butyric acid in stool extract is by liquid chromatography with mass spectrometry, preferably tandem mass spectrometry.

In some embodiments, the biogenic amines and neuroactive substances of the gut-brain-axis are collectively subjected to derivatization prior determination. The method of diagnosis comprises predetermined cut-off values for tryptophan, serotonin and gamma-amino butyric acid in stool determined in comparison with values found in stool specimens of healthy subjects.

In some embodiments, the stool extract of neuroactive substance of the gut-brain axis is further analyzed for its content of kynurenic acid, and as a supportive biomarker, for its content of tissue-active histamine.

The combined cut-off values in stool for in-vitro diagnosis of irritable bowel syndrome or irritable colon are partly dependent on the method of extraction but they should be in the range of 15 to 25 [µg/g] tryptophan, 700 to 2500 [ng/g] serotonin and 10-15 [µg/g] gamma-amino butyric acid, 1.5 to 2.5 [µg/g] kynurenic acid, preferably 18.4 [µg/g] tryptophan, 740 to 2500 [ng/g] serotonin, 13.6 [µg/g] gamma-amino butyric acid and 1.9 [µg/g] kynurenic acid.

Another aspect relates to a kit for in-vitro diagnosis of irritable bowel syndrome or irritable colon, comprising: -
a stool sample extraction system for taking a defined stool specimen and preparation of an extract of active substances of the gut-brain-axis from stool in a stabilizing buffer;
a derivatizing reagent which react with small biogenic amines;
antibodies specific for derivatized tryptophan, serotonin and GABA; and
   i mmunoassay reagents and buffers for combined determination of tryptophan, serotonin and GABA and for comparison with the combined threshold values of healthy controls.

In some embodiments, the kit may also contain the reagents for immunological determination of histamine and kynurenic acid.

### DETAILED DESCRIPTION OF PREFERRED EMBODIMENTS

The invention relates to a diagnosis, dietetic treatment and/or pain relief caused by gastrointestinal disorders in subjects suffering from irritable colon or irritable bowel syndrome. To date, there is no effective therapy that has been tested in clinical studies. Dietary fibers and Mebeverine®, an anticholinergic are used to alleviate some of the symptoms of irritable bowel syndrome (IBS) and related conditions; specifically stomach pain and cramps, persistent diarrhea, and flatulence. Mebeverine® appears to work directly on the smooth muscle within the gastrointestinal tract and may also have an anesthetic effect. Data from controlled clinical trials have however not found a difference from placebo in alleviating stomach pain in people suffering from irritable colon or irritable bowel syndrome (Annaházi A et al. in Role of antispasmodics in the treatment of irritable bowel syndrome". World Journal of Gastroenterology 2014, 20(20):6031-43; Darvish-Damavandi M et al., in A systematic review of efficacy and tolerability of mebeverine in irritable bowel syndrome". Gastroenterology. 2010, 16(5): 547-53)

Irritable bowel syndrome or irritable colon is currently based on an exclusion diagnosis when the symptoms in the lower abdomen and changes in stool frequency are not caused by organic disturbances such as ulcers or biochemical abnormalities and when these symptoms last longer than three months. This does not imply that the symptoms are harmless and mean nothing to those affected. The quality of life can be significantly impaired. Irritable bowel syndrome rarely comes alone. Frequently, reflux symptoms and retrosternal burning occur at the same time. Extraintestinal symptoms include migraine, back pain and fibromyalgia. Irritable bowel syndrome is an ordeal not only because of its agonizing symptoms. Physical causes cannot be determined, patients are not taken seriously, and doctors are helpless. For a long time, the disease was regarded as a psychosomatic illness without an organic basis. Today, more is known about the complex network of physiological and psychological factors that determine the clinical picture. To date, there are no means for positive diagnosis.

The present inventor has discovered that irritable bowel syndrome and irritable colon can be diagnosed by examining the neuroactive substance in stool, notably the tryptophan-derived agonistic and antagonistic messengers in stool. The sensitive and motor functions of the intestines are regulated by complex interactions which has so far escaped description. On the other hand, the enteric, vegetative and central nervous systems are linked to each other in many ways. In irritable bowel syndrome, this complex regulation is disturbed which becomes particularly evident in an altered intestinal motility: It can be increased or decreased, which is the case in about one third of all patients. In addition to irritable bowel patients of the diarrhea type and those of the constipation type, there is a third group with mixed forms. The physiological axis of the microbiome, gut/colon and the brain is up- and down regulated by a sizeable number of neurotransmitters while the symptoms of the gastrointestinal disorders are manifold and complex.

The pain suffered by patients with irritable bowel syndrome only partly indicates changes in motility. Studies of colon stretching showed that patients with irritable bowel syndrome have a significantly lower threshold for abdominal stretch compared to healthy normal subjects. When a balloon was stretched in the colon, irritable bowel patients already perceived volumes as unpleasant that did not trigger any stimuli at all in normal subjects. Based on these studies, experts formulated the theory of "visceral hypersensitivity". According to this theory, the perception of pain stimuli in the intestine in patients with irritable bowel syndrome is pathologically increased. The serotonin receptors play a central role in mediating pain stimuli and changes in intestinal motility and secretion. Excessively released serotonin, coupled with a hypersensitivity of the receptors of subtype 5-HT3 in the intestinal wall, lowers on the one hand the perception threshold for pain stimuli. On the other hand, it increases the motility of the intestinal muscles and the secretion of mucus. Another important receptor subtype are the 5-HT4 receptors. If serotonin binds to these receptors, intestinal peristalsis as well as water and electrolyte release are stimulated. These effects are highly desired in irritable bowel patients of the constipation type. More than 90% of the body's serotonin is synthesized in the gut, where 5-HT activates gut motility via the 5-HT3-receptor on enteric neurons. Low 5-HT levels in the gut, colon and stool may therefore lead to constipation (IBS-C). Very high levels of serotonin on the other hand may cause diarrhea (IBS-D).

The ultimate goal for treatment of irritable bowel syndrome is to provide relief for the multiple symptoms of this condition. This cannot be achieved by a single drug or drug therapy which can only alleviate some of the symptoms nor represent a therapy. The prospect of a long-term treatment efficacy is therefore limited as current therapies are not curative. Probiotics are commonly prescribed because dysbiosis has been suggested to be part of the multifactorial etiology of irritable bowel syndromes and irritable colon. However, despite of numerous studies and meta-analyses, no firm conclusions can be drawn from the efficacy of probiotics. Strong placebo effects, psychological factors, and gender effects make the interpretation of findings difficult and may disguise the true effects of probiotic products if existent at all. On the other hand, the gut microflora has been shown to significantly influence microglia from before birth until adulthood, and that the metabolites generated by the gut microbiota may regulate not only the inflammation response but impact the gut-brain axis (Thion MS et al, in Microbiome Influences Prenatal and Adult Microglia in a Sex-Specific Manner, Cell 2018,172(3):500-516) In detail, the gut microbiota has been shown to regulate the biosynthesis of host serotonin which can affect the hosts' behaviour (Yano JM et al in Indigenous Bacteria from the Gut Microbiota regulate Host Serotonin Biosynthesis, Cell 2015, 161(2):264-76) as well as the intestinal motility. The present inventor has discovered that a combined quantitative determination of gamma-aminobutyric acid, serotonin, and L-tryptophan in stool allows a positive diagnosis of irritable colon and/or IBS and that a combined quantitative evaluation of these biomarkers in stool can give way to founded dietary instructions which address the etiology of irritable bowel syndromes and ease the gastrointestinal symptoms.

Without being bound by a theory the stated neuroactive biomarkers describe, if looked at in combination, the chemical signaling between the community of bacteria and microglial cells in the gut. The microglial cells in the gut contribute and regulate the pain conditions of irritable colon and irritable bowel syndrome. While the understanding of the development and function of microglia is incomplete, especially regarding the factors affecting microglial function, there is evidence that the microbiota influences the microglia in the gut and so the generation of certain chronic pains. The instant disclosure provides a strategy for reducing microglial activation and the generation of pains which are caused by an inflammation or infection. A combined measurement of the stated biomarkers in stool gives also way to a targeted dietetic manipulation of the gut microbiome and to a viable strategy in lessening the pains in irritable bowel syndromes. As increased microglial activation and neural inflammation can also be held responsible for certain cases of sustained hypertension, the inventor further contemplated that the present disclosure gives also way to a dietetic treatment of this condition (Sharma RK et al in Microglial Cells Impact Gut Microbiota and Gut Pathology in Angiotensin II-Induced Hypertension, Circulation Res. 2019, 124:727-736). Consequently, a knowledge of the network of the neuroactive tryptophan-related biomarkers between the microbiota and the microglia in the gut will allow dietetic recommendations for a supportive and temporary treatment or even a full cure of irritable bowel syndrome and irritable colon. It is surprising that extracts of solid or soft stool are "consistent samples" for the neuroactive messengers of the gut-brain-axis.

Patients suffering from irritable bowel syndrome often show an elevated proportion of *Firmicute's* and a reduced proportion of *Bacteroides* in stool. A raised F/B ratio can be correlated with an increased body mass index (BMI) as well as with depression (Kelly JR et al, Breaking down the barriers: the gut microbiome, intestinal permeability and stress related psychiatric disorders, Front Cell Neurosci. 2015, 9: 392f). A raised F/B ratio impacts also the neuroactive messengers between the gut microbiome and the central nervous system (CNS) and correlates with stress-related psychiatric disorders as well as with the core symptoms of irritable bowel syndrome (Saulnier DM et al., Gastrointestinal Microbiome Signatures of Pediatric Patients with irritable Bowel Syndrome, Gastroenterology 2011, 141; Swidsinski, A et al., Spatial Organization and Composition of the Mucosal Flora in Patients with Inflammatory Bowel Disease, J Clin Microbiology 2005, 43; Zhuang X1, Alterations of gut microbiota in patients with irritable bowel syndrome: A systematic review and metaanalysis. J Gastroenterology & Hepatology 2016, 10). A genetic typing of the bacterial phyla in the gut however allows no diagnosis of irritable bowel syndrome. The bacterial phyla can only be statistically associated with symptoms but not be used for diagnosis or treatment of the pains associated with irritable bowel syndrome. This is because the pathophysiology of pain and chronic pain encompasses complex sensory, immune, and inflammatory interactions within both the central and peripheral nervous systems. Notwithstanding, it appears obvious that the gut resident cells and the macroglia are critically involved and that they respond to both signals from the CNS as well as to "arbitrary" signals from the microbiome. However, many factors can alter the composition of the gut microbiota, including infection, antibiotic medication, nutrition and food, as well as environmental stressors, and host genetics. Stress can significantly impact the microbiota-gut-brain axis at all stages of life. The gut microbiota however is primarily determined by the daily food intake and eating habits and the F/B ratio can be impacted by dietetic measures and manipulation. A targeted manipulation of the gut microbiota however requires suitable biomarkers. The chemical communication between the gut microbiome and the microglia as well as within the gut brain axis will now be discussed by way of examples and neuroactive biomarkers detectable and quantifiable in stool extracts: -

### EXAMPLES

### Example 1 L-Tryptophan

L-Tryptophan is an essential amino acid which must be derived from the diet. Apart from its role in protein synthesis, L-tryptophan is a precursor of serotonin, a number of indole derivatives with anti-inflammatory effect (Zelante T et al. in Tryptophan Catabolites from Microbiota Engage Aryl Hydrocarbon Receptor and Balance Mucosal Reactivity via Interleukin-22, Immunity 2013, 39(2):372-385) and various signaling molecules in the gut-brain communication. The brain seems to communicate to some extent via the tryptophan metabolism. Tryptophan is further involved in the activation of repair processes in the intestinal mucosa and activates inter alia mTOR, which leads to an increased formation of barrier proteins, defensins and secretory IgA (Liang H et al. in Dietary L-Tryptophan Supplementation Enhances the Intestinal Mucosal Barrier Function in Weaned Piglets: Implication of Tryptophan-Metabolizing Microbiota, Int. J. Mol. Sciences 2018, 20(1):20). Humans metabolize about 90% of the L-tryptophan in food to kynurenine and approximately 3 to 5% to serotonin. The remaining 7% is degraded by the gut microbiota to indole and its derivatives. An intestinal inflammation however redirects the tryptophan metabolic pathways towards kynurenic acid compared to serotonin (Gupta NK et al. in Serum Analysis of Tryptophan Catabolism Pathway: Correlation with Crohn's Disease Activity, Inflammatory Bowel Diseases 2012, 18 (7):1214-20). On the other hand, there is a negative correlation between serum levels of tryptophan and IBD activity.

The L-tryptophan concentration in stool is therefore a useful biomarker for a diagnosis of irritable bowel syndrome and irritable colon, if measured in combination with neuroactive metabolics. When the L-tryptophan concentration in stool is higher than 18.4 µg/g stool (90 nmol Trp/g stool) patients have no symptoms of irritable colon or irritable bowel syndrome.

### Example 2 Serotonin (5-Hydroxytryptophan (5-HT)

5-Hydroxytryptophan (5-HT or serotonin) is a metabolic of tryptophan and produced by the gut microbiome as well. Serotonin is also contained in many foods. Walnuts may contain over 300 µg/g of serotonin but likewise many vegetables, fruits and fungi. More than 1µg/g may be contained in bananas, chicory, Chinese cabbage, cocoa coffee, green coffee bean, green onion, hazelnut, kiwi, lettuce, nettle, Griffonia simplicifolia, paprika, passion fruit, pawpaw, pepper, pineapple, plantain, plum, pomegranate, potato, spinach, strawberry, tomato, velvet bean, wild rice, and products derived from them such as chocolate. Serotonin is known to control or modulate gut motility, secretory reflexes, platelet aggregation, regulation of immune responses, as well as mood and behavior.

In humans about 95% of the total serotonin is found in the gastrointestinal tract and about 90% thereof in enterochromaffin cells (Kim DY et al, Serotonin: a mediator of the brain-gut connection, in Am J Gastroenterol. 2000, 95(10):2698-2709). The serotonin in stool is increased as a reaction to chemical stimuli or stretch stimuli. High concentrations of serotonin in stool can be correlated with increased gut motility and diarrhea, low concentrations with constipation (Dunlop et al. in Abnormalities of 5-hydroxytryptamine metabolism in irritable bowel syndrome, Clinical Gastroenterology and Hepatology 2005, 3(4):349-357; Gershon MD in 5-Hydroxytryptamine (serotonin) in the gastrointestinal tract, Curr Opin Endocrinol Diabetes Obes 2013, 20, 14-21; Gershon MD in Serotonin is a sword and a shield of the bowel: serotonin plays offense and defense, Transactions of the American Clinical and Climatological Association 2012, 123: 268-80; Badawy A et al in Tryptophan: the Key to Boosting Brain Serotonin Synthesis in Depressive Illness. J Psychopharmacology 2013, 27:878-93; Reigstad CS et al., Gut microbes promote colonic serotonin production through an effect of short-chain fatty acids on enterochromaffin cells. The FASEB Journal 2015, 29:1395-1403; Horii Y et al. in The serotonin receptor mediates changes in autonomic neurotransmission and gastrointestinal transit induced by heat-killed Lactobacillus brevis SBC8803, Beneficial Microbes 2015, 6(6):817-822).

Upon local release by enterochromaffin cells the serotonin in stool seems to mediate also the development of abdominal pain and increased intestinal motor function. An increase of the serotonin levels can be obtained by the administration of selective serotonin reuptake inhibitors (SSRIs) which are usually prescribed as antidepressants. As the name suggests, they inhibit the reuptake of serotonin into the storage vesicles. This allows the serotonin to develop its effect as a neuroactive messenger for longer. Well-known active substances in the SSRI group include citalopram, paroxetine and fluoxetine. Patients often receive also serotonin reuptake inhibitors which additionally inhibit the uptake of noradrenalin (SSNRI). Representatives of the SSNRI group are venlafaxine and duloxetine. Common side effects of these drugs are restlessness, headache and nausea. However, there is no diagnostic available whether a up or down-regulation of serotonin and its activities is needed. Drugs for treatment of irritable bowel syndrome which attack the serotonin receptors are the selective 5-HT3 receptor antagonist Alosetron (Lotronex®) and the partial 5-HT4 agonist Tegaserod (Zelmac®).

The present application discloses that patients are not suffering from symptoms of irritable colon and irritable bowel syndrome when tryptophan levels in stool are higher than 90 nmol tryptophan /g stool (18.4 µg/g) and serotonin levels in stool in the range from 4.2 to 14.2 nmol/g stool (740 to 2.500 ng serotonin/g stool). When the tryptophan metabolisms by either microbiome or host are disturbed in any way, as measurable by the combination of both stool biomarkers, patients seem to suffer from diffuse abdominal pain and/or altered stool frequency and consistency. Recently, some new studies claim that the microbiome communicates with cells producing serotonin and that certain gut bacteria can worsen metabolism and/or signal to cells in the gut to produce more serotonin (Fung TC et al. in Intestinal serotonin and fluoxetine exposure modulate bacterial colonization in the gut. Nature Microbiology, 2019; DOI: 10.1038/s41564-019-0540-4). However, more research will be needed to elucidate the underlying mechanisms and physiological effects.

### Example 3 γ-Aminobutyric acid (GABA)

A major source of γ-aminobutyric acid (GABA) in stool is the enzymatic conversion of L-glutamate by glutamate decarboxylase, again by both gut microbiome and host. *Lactobacillus* and *Bifidobacterium* species are examples of GABA-producing bacteria in the gut. GABA is further contained in many vegetables and foods such as Adzuki bean, barley, broccoli, buckwheat, chestnut, common bean, kale, lupin, maypop, mouse-ear hawkweed, oat, pea, pokeroot, potato, rice, shiitake, soya bean, spinach, St John's wort, sweet potato, tea, tomato, valerian, wheat, wild celery. Fruits of tomato also contain a substantial amount of GABA during the mature green stage. Enzymatic or whole-cell biocatalysts and microbial fermentation are further employed to generate substantial amounts of GABA in foods, e.g. in soya yogurt, black raspberry juice, GABA-enriched supplements, because of the potential health benefits of GABA.

GABA is a major inhibitory neurotransmitter and regulates various processes in peripheral organs, including the colon. GABA exerts its functional effects via two GABA receptor subclasses - GABA_{A} and GABA_{B}. GABA is known having analgesic effects and anti-anxiety and hypotensive activity, the latter being used for promoting stress tolerance, relief of symptoms of mental stress and as a sleep aid. GABA is taken up in the gut by presynaptic neurons so that GABA in the gut is assumed modulating the excitability of colonic sensory afferents. It can therefore be assumed that the neuromodulatory properties of microbial-derived GABA and the GABAergic signaling via the microbiome-gut-brain axis have a role in the development of constipation and visceral pain.

Therapeutic interventions include (i) the oral administration of GABA or Gabapentin (Neurontin®), an anticonvulsant medication only FDA-approved for treatment of focal and mixed seizures, and/or (ii) the oral administration of GABA producing bacteria such as Lactobacilli and Bifidobacterium and/or (iii) dietetic intervention. Low GABA concentrations in stool increases visceral sensitivity. If the concentrations of tryptophan in stool is higher than 90 nmol/g stool and serotonin in the range from 4.2 - 14.2 nmol/g stool, a γ-aminobutyric acid (GABA) concentration in stool higher than 132 nmol GABA/g stool (13.6 µg GABA/g stool) indicates a balanced ratio of neuroactive substances in the gut.

### Example 4 Kynurenic acid (KynA)

Kynurenic acid (KynA) is a further metabolic of tryptophan and acts as an antiexcitotoxic and anticonvulsant in the gut-brain axis. It is well known that nerve cells will be damaged by excessive stimulation through neurotransmitters such as glutamate, N-methyl-D-aspartic acid (NMDA) or α-amino-3-hydroxy-5-methyl-4-isoxazolepropionic acid (AMPA). These neurotransmitters are however sometime generated by some prokaryotes in the gut. NMDA in subtoxic amounts induces neuronal survival but excess glutamate is toxic as it allows high levels of Ca²⁺ influx into cells which activates phospholipases, endonucleases, and proteases, e.g. calpain, and these enzymes can damage cell structures, membranes and components of the cytoskeleton. In evolved adaptive systems as in the gut, the damaging mechanisms are rarely, if ever, simplistically direct but KynA in stool can act as an anticonvulsant by blocking the sodium channels or by enhancing the GABA function. Next to the voltage-gated sodium channels and components of the GABA system, the targets may also include GABA receptors, the GAT-1 GABA transporter, and GABA transaminase. By blocking sodium or calcium channels, KynA may reduce the effects of excitatory glutamate, NMDA and AMPA since it is a known antagonist at ionotropic AMPA, NMDA and kainic acid receptors. KynA is further a noncompetitive antagonist at the glycine site of the NMDA receptor and an antagonist of the nicotinic acetylcholine receptor.

The inventors therefore contemplate not only to measure the concentrations of tryptophan, serotonin and GABA in stool but also of KynA for its antagonistic properties to excitatory neurotransmitters. A kynurenic acid (KynA) concentration of higher than 10 nmol KynA/g stool (1.9 µg KynA / g stool) is regarded a supportive biomarker to exclude diagnosis of irritable bowel syndrome.

### Example 5 Histamine

Histamine is a tissue hormone that acts in the gut on mast cells, gastric enterochromaffin-like cells and nerve cells and impacts immunomodulatory processes such as the chemotaxis of eosinophilic and neutrophilic cells as well as the production of prostaglandins and cytokines (e.g. IL-6 or TNFα). Histamine may also be produced by some bacteria in the gut and is contained in many foods and drinks, such as aged cheeses, red, white and dessert wines, ham and other cured dry meat products, Anchovy, beer, billfish, Champagne and Sherry, nettle, dandelion, fermented sausages, sauerkraut, fish (herring, sardine, tuna, mackerel, and bonitos) ketchup, soybean food products (e.g. soy, tempeh, soy sauce, soya bean milk, doenjang, doufuru, and natto̅), sweet or sour cream, yoghurt, pasteurized, and fresh milk. When there is imbalance between histamine intake and histamine degradation, or a histamine producing microbiota the high intestinal histamine levels can lead to nausea, headaches, dyspnea, tachycardia and diarrhea (IBS-D) (Smolinska S et al. in Histamine and gut mucosal immune regulation, Allergy 2014, 69(3):273-281; Kovacova-Hanuskova E et al. in Histamine, histamine intoxication and intolerance, Allergologia et Immunopathologia 2015, 43(5):498-506). Histamine concentrations in stool should be lower than 900 ng/ml.

It is however questionable, whether a deficiency of diaminooxidase or intestinal problems in the following of an excessive intake of histamine containing food can be considered irritable bowel disease or a separate medical entity despite the similar pains and symptoms. Notwithstanding, a determination of histamine is a good companion marker when testing a stool sample for a diagnosis of irritable bowel disease or irritable colon.

### Example 6 Analysis of stools of IBS patients for active substance of the gut-brain axis

40 Fecal samples (stools) from patients were tested in combination for tryptophan, serotonin, GABA and KynA. The patients were suffering from functional gastrointestinal disorders and were diagnosed or strongly suspected of suffering from irritable bowel syndrome, say irritable bowel syndrome (IBS) and constipation (IBS-C) or diarrhea (IBS-D) or IBS-M (mixed). A subset of patients (30-40%) thereof further reported to experience enhanced sensitivity to colonic distension, accompanied by a reduced threshold for pain and increased intensity of sensation. Visceral hypersensitivity was therefore further an inclusion criterium. The cause of visceral hypersensitivity is unknown, but the recurrent abdominal pain attributed to visceral hypersensitivity is a common and costly health-care problem. The amplification of visceral signals to the brain may occur at various levels, including at the level of the intestinal mucosa. Responses of these visceral afferents are likewise affected by local chemical and mechanical stimuli but may also be influenced by interactions with the intestinal microbiota and therefore this condition was considered indistinguishable from IBS. It can therefore be assumed that the samples were representative for the patients seeking dietary advice because of suffering from the various forms of irritable bowel syndrome and an imbalance of neuroactive substance and transmitters in the gut.

15 mg stool was extracted in 0.75 mL Amino Extract stool extraction buffer (50 mM citrate buffer) using the IDK® stool extract system (Immundiagnostik AG, Bensheim, DE-Art. No. K6998SAS) for 15 minutes. Particulate material in the extract was removed either by centrifugation or sedimentation. The supernatant was used for derivatization and immunological analysis.

Derivatization of Trp-Sero-GABA: 50 µl stool extract was diluted in 500 µL 25 mM phosphate buffer (pH 8,0) and derivatized using a stoichiometric excess of derivatizing reagent, more precisely by 25 µL 25 mM/L Boc-Ahx-Trp-β-Ala-NHS in DMSO for 30 minutes as described in EP 2612147 B1. After combined derivatization of the neuroactive biogenic amines in stool (tryptophan, serotonin, gamma-amino butyric acid), the reaction was stopped by the addition of 1% BSA-solution and the derivatized sample subjected to competitive immunoassays. Commercial immunoassays can be used for determination of the neuroactive substances in stool, namely a IDK® tryptophan ELISA (Immundiagnostik AG, Bensheim, DE-Art.No. K7729), IDK® GABA ELISA (Immundiagnostik AG, Bensheim, DE - Art.No. K7009) and IDK® serotonin ELISA (ImmundiagnostikAG, Bensheim, DE-Art.No. K6881). Histamine can be determined using the IDK® histamine ELISA (Immundiagnostik AG, Bensheim, DE - Art.No. K8213). L-kynurenic acid was determined using a novel competitive immunoassay (ELISA) employing a polyclonal antibody against L-kynurenic acid conjugated to KLH as immunogen. The assay is based on the method of competitive enzyme linked immunoassays analog to IDK® kynurenic acid ELISA (Immundiagnostik AG, Bensheim, DE - Art.No. K7735). Samples, standards and controls were incubated in wells of a streptavidin- microtiter plate coated with kynurenic acid conjugated to biotin (antigen), together with a peroxidase labeled polyclonal anti-kynurenic acid antibody. During the incubation period the free target antigen in the stool extract competes with the antigen immobilized on the wall of the microtiter wells for the binding of the peroxidase-labelled polyclonal antibodies. After a washing step to remove the unbound components, the peroxidase substrate tetramethylbenzidine (TMB) is added. Finally, the enzymatic reaction is terminated by an acidic stop solution. The color changes from blue to yellow and the absorbance is measured in the photometer at 450 nm. The intensity of the yellow color is inverse proportional to the kynurenic acid concentration in the sample; this means, high antigen concentration in the sample reduces the concentration of antibodies bound to the antigen on the plate and lowers the photometric signal. A dose response curve of the absorbance unit (optical density, OD at 450 nm) vs. concentration is generated, using the values obtained from the standards. Kynurenic acid, present in the patient samples, is determined directly from this curve.

The results have been summarized in TABLE 1 below.

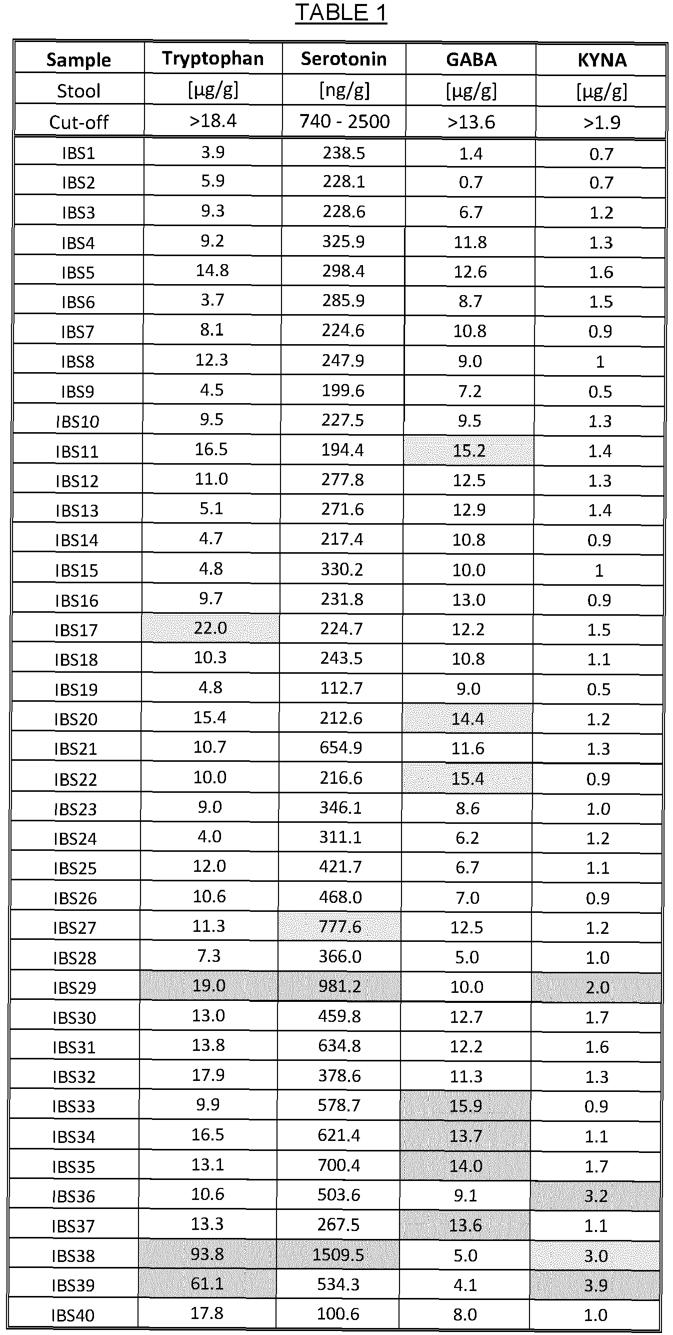

The table shows that in the group of 40 diagnosed IBS patients 90% (36 subjects) showed an imbalanced serotonin-tryptophan ratio or a substantive deficiency of either tryptophan or its metabolic serotonin in the stool, and therefore in the gut as well. 82.5% of the IBS patients (4) were deficient of inhibitory neurotransmitter GABA in stool and/or gut. 74% of the IBS patients had a deficiency of three neuroactive substances of the gut-brain-axis (Trp-Sero-GABA). A positive diagnosis of IBS - which was so far only possible by exclusion diagnostics - can be statistically further supported by having KynA measured in stool since more than 90% of the IBS patients showed a deficiency of KynA in stool. If KynA is added as stool biomarker, 85% of the IBS patients showed in addition a deficiency of all three KynA-tryptophan-serotonin stool biomarkers. Consequently, the given biomarkers in stool allow for reliable *in-vitro* diagnosis of IBS and irritable colon. In addition, a diagnosis as described further allows dietary recommendation and treatment of the symptoms of IBS and irritable colon since the correlation between the various deficiencies and intestinal health is obvious.

For comparison, none of the stools of 40 healthy subjects showed any signs of an imbalanced serotonin-tryptophan-ratio and/or a deficiency in kynurenic acid or GABA.

## Claims

1. Method of *in vitro* diagnosis of irritable bowel syndrome (IBS) or irritable colon, comprising the steps of:-
collecting a quantitatively defined specimen of fresh stool;
transferring the stool specimen into a stool stabilizing buffer;
extracting the stool for neurotransmitters, neuroactive substances and tissue-active substances to produce a solution of neuroactive substances of the gut-brain axis;
analyzing the solution of neuroactive substance of the gut-brain axis in combination for the amounts of tryptophan, serotonin and gamma-amino butyric acid;
to determine any imbalance of neuroactive substances in stool for diagnosis of irritable bowel syndrome and/or irritable colon on the basis of cut-off-values or ranges for three or more neuroactive substances.

2. Method as claimed in claim 1, wherein the combined determination of tryptophan, serotonin and gamma-amino butyric acid in stool extract is by immunoassays.

3. Method as claimed in claim 1, wherein the combined determination of tryptophan, serotonin and gamma-amino butyric acid in stool extract is by liquid chromatography with mass spectrometry, preferably tandem mass spectrometry.

4. Method as claimed in any claim 1 to 3, wherein the biogenic amines and neuroactive substances of the gut-brain-axis are collectively subjected to derivatization prior determination.

5. Method as claimed in any claim 1 to 4, comprising predetermined cut-off values for tryptophan, serotonin and gamma-amino butyric acid in stool.

6. Method as claimed in any claim 1 to 5, wherein the stool extract of neuroactive substance of the gut-brain axis is further analyzed for its content of kynurenic acid.

7. Method as claimed in any claim 1 to 6, wherein the stool extract is further analyzed for its content of tissue-active histamine.

8. Method as claimed in claim 5, wherein the combined cut-off values in stool for in-vitro diagnosis of irritable bowel syndrome or irritable colon are in the range of 15 to 25 [µg/g] tryptophan, 700 to 2500 [ng/g] serotonin and 10-15 [µg/g] gamma-amino butyric acid, 1.5 to 2,5 [µg/g] kynurenic acid, preferably 18.4 [µg/g] tryptophan, 740 to 2500 [ng/g] serotonin, 13.6 [µg/g] gamma-amino butyric acid and 1.9 [µg/g] kynurenic acid.

9. Kit for *in-vitro* diagnosis of irritable bowel syndrome or irritable colon, comprising:-
a stool sample extraction system for taking a defined stool specimen and preparation of an extract of active substances of the gut-brain-axis from stool in a stabilizing buffer;
a derivatizing reagent which reacts with small biogenic amines;
antibodies specific for derivatized tryptophan, serotonin and GABA; and
. immunoassay reagents and buffers for combined determination of tryptophan, serotonin and GABA and for comparison with the combined threshold values of healthy controls.

10. Kit for *in-vitro* diagnosis of irritable bowel syndrome or irritable colon, further comprising reagents for immunological determination of histamine and kynurenic acid.
